# EUROPEAN PATENT APPLICATION

(11) **EP 1 523 966 A1**
(43) Date of publication of application: **20.04.2005**
(21) Application number: 04105051.9
(22) Date of filing: 14.10.2004
(51) Int. Cl.: A61F 5/37

(54) **A restraining device**

(30) Priority: 14.10.2003 SE 3027083
(71) Applicant: Careva Systems Aktiebolag, S-417 55 Göteborg (SE)
(72) Inventor: BJÖRK, Eva-Stina, 412 53, GÖTEBORG (SE)
(74) Representative: Johansson, Lars E.

(57) **Abstract**

The invention relates to a device for providing posture support to a seated user (P). The device comprises a seat pad (1) with a top surface (2) and a bottom surface (3). At least a part of the bottom surface (3) is a surface (3) with high friction to prevent slipping. A first belt (4) is attached to the seat pad and adapted to locked into a loop such that the first belt can loop a part of the user's body. Thereby, the belt can secure the rear of the user to the seat pad (1).

## Description

### FIELD OF THE INVENTION

The present invention relates to a device for providing posture support to a seated user, a seat provided with a device for posture support, a vehicle provided with such a seat and a method for securing a physically impaired person in a seat.

### BACKGROUND OF THE INVENTION

For individuals who are physically impaired, sitting upright in a chair or on a vehicle seat can be difficult. This can be, for example, individuals with reduced muscle strength. This can also be the case for individuals with certain neurological disorders, for example certain hyperactive individuals who are unable to fully control their own movements. For such individuals, it may be necessary to use a support device that helps them to maintain a correct seated position. It has been proposed that such individuals can be held in position by means of a belt or a body harness that secures the user to the seat or chair. To be effective, such a device should preferably prevent the user from falling or bending forwards or to the sides. It is also desirable that the user is prevented from sliding against the seat cushion such that the user no longer remains in an upright position. In the prior art, it has been suggested that a belt around the user's waist should hold the user against the backrest/seatback of the seat or chair. However, the inventor of the present invention had found that the lower part of a user's body can still slide against the seat cushion such that an upright position cannot be maintained. To prevent the user's body from falling forwards, it has been suggested to use belts that hold the upper part of the user's torso against the backrest. If such a belt is tightened over the user's chest, this may be uncomfortable for certain user's, in particular women. For increased comfort, such belts can be crossed over the user's chest. However, it is then important that such a crossed belt will not cause reduced comfort for other reasons. Therefore, it is an object of the present invention to provide a posture support device that effectively prevents the user from sliding against the seat cushion and is comfortable and easy to use.

### DESCRIPTION OF THE INVENTION

The invention relates to a device for providing posture support to a seated user. The device comprises a seat pad with a top surface and a bottom surface. The inventive device is provided with means for counteracting or preventing sliding of the seat pad. In a preferred embodiment of the invention, at least a part of the bottom surface is a surface with high friction. Optionally, or as an alternative to using a high friction surface, a strap or other fastening means can be attached to the chair and to the bottom part of the pad to secure the pad against sliding. A first belt is attached to or attachable to the seat pad. The first belt is adapted to be locked into a loop such that the first belt can loop a part of the user's body when the user is sitting on the top surface of the seat pad and thereby secure the rear of the user to the seat pad.

Preferably, the device also comprises a second belt that can be fastened to the backrest of a seat or chair, the device further being adapted to fasten ends of the second belt either to the first belt or to the seat pad or to the cushion of a seat such that the second belt thereby can secure the user to the backrest of the seat or chair. In an advantageous embodiment, the first belt is provided with straps. The straps and the ends of the second belt may have cooperating fasteners such that the ends of the second belt can be locked to the straps.

Preferably, the device additionally comprises a third belt having means for securing the third belt to the backrest of a seat or chair. The third belt is formed with at least one channel through which the second belt can be passed and thereby secured to the backrest. It should be understood that the third belt could comprise a plurality of channels placed above each other. In this way, the second belt can be secured to the backrest at different vertical levels when the third belt is secured to the backrest of a seat or chair.

In a preferred embodiment of the invention, the at least one channel is provided with a stiffening insert/element. Such a stiffening insert or stiffening element serves to prevent the third belt from curving around the neck of a user when the ends of the second belt are fastened to the first belt or the seat pad. It is also possible to use other means for preventing the belt or belts from curving around the neck of the user.

Preferably, the lengths of the first belt and the second belt can be adjusted.

The invention also relates to a seat or chair having a cushion and a first belt with adjustable length and adapted to be locked over a part of the body of a user/person sitting on the seat or chair such that the first belt locks over the body below the hips of the person sitting on the seat or chair, whereby tightening of the first belt will secure the rear of the person's body against the cushion of the seat or chair.

In a preferred embodiment of the invention, the seat or chair additionally comprises a backrest and second belt means fastened to the backrest. The second belt means is adapted to be fastened to either the cushion or to the first belt such that the torso of a user/person sitting in the seat or chair is secured against the backrest. The second belt means could comprise a belt that is removably attached to the backrest of the seat or chair. Alternatively, the second belt means could comprise straps/belts that are integral with the backrest.

The invention also relates to a vehicle comprising at least one vehicle seat shaped according to the invention or being equipped with a posture support device according to the invention.

### BRIEF DESCRIPTION OF THE DRAWINGS

- Figure 1: shows one side of a seat pad with a first belt for locking the seat pad to the body of a user.
- Figure 2: shows the opposite side of the seat pad shown in Figure 1.
- Figure 3: is a side view of the seat pat of Figure 1 and Figure 2.
- Figure 4: shows a second belt for securing and supporting a user/person against a backrest of a seat or chair.
- Figure 5: shows the belt of Figure 4 where the ends of the belt have been crossed in a way suitable for securing a seated user against a backrest.
- Figure 6: is a front view of a third belt that can be secures to the backrest of a seat and used for securing the second belt to the backrest.
- Figure 7: is a side view of the belt shown in Figure 6.
- Figure 8: is an enlargement of a section of Figure 7.
- Figure 9: shows how the components of the inventive posture support device can be connected to each other.
- Figure 10: shows the inventive posture support device applied to a seat or chair.
- Figure 11: shows the back of a seat with the third belt secured to the backrest.
- Figure 12: is a front view of a seated user wearing the inventive posture support device.
- Figure 13: is a side view of a seated user wearing the inventive posture support device.
- Figure 14: shows an alternative embodiment of the invention.
- Figure 15: shows yet another alternative embodiment of the invention.
- Figure 16: shows an additional embodiment of the invention.
- Figure 17: is a view from behind of the embodiment showed in Figure 16.

### DETAILED DESCRIPTION OF THE INVENTION

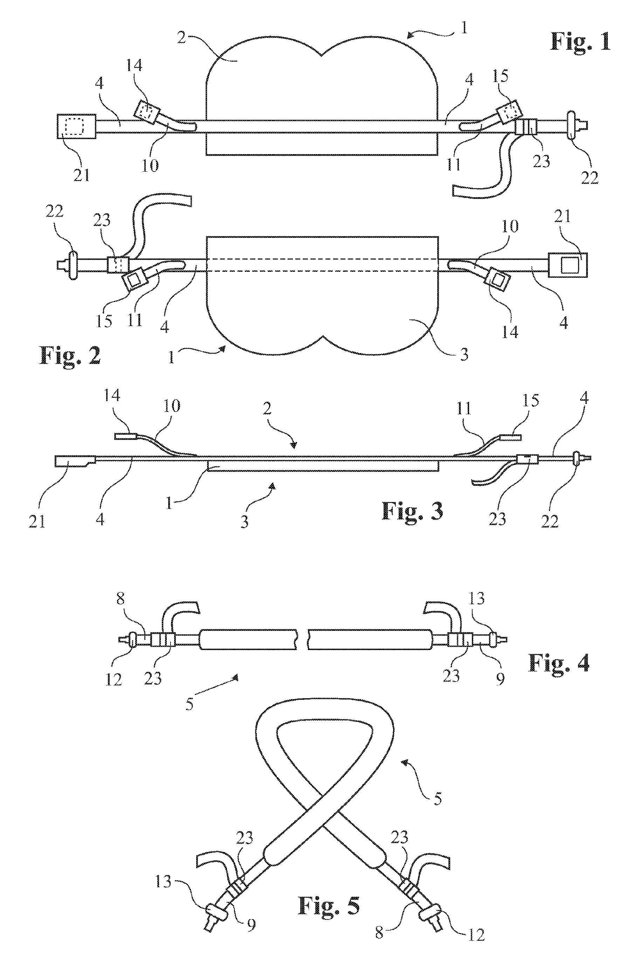

With reference to Figures 1-3, the inventive posture support device comprises a seat pad 1 with a top surface 2 and a bottom surface 3. In preferred embodiments of the invention, the device comprises means for counteracting or preventing sliding of the seat pad 1. In one advantageous embodiment, at least a part of the bottom surface 3 is a surface 3 with high friction. Preferably, the entire bottom surface or essentially the entire bottom surface 3 is a surface 3 with high friction. A possible material for the friction surface 3 may be, for example, foamed plastic. In an advantageous embodiment of the invention, at least a part of the top surface 2 is also a surface with high friction. It is also conceivable that the entire top surface 2 is a surface with high friction. A first belt 4 is attached to or attachable to the seat pad 1. The first belt 4 is adapted to be locked into a loop such that the first belt can loop a part of the body of a user (a person using/wearing the posture support device) when the user is sitting on the top surface of the seat pad 1 and thereby secure the rear of the user to the seat pad 1. In the figures, the first belt 4 is showed with fasteners such as buckles 21, 22 known per se. For example, the buckles 21, 22 could be of the same type used for ordinary seat belts. The length of the belt 4 can be adjusted by means of a tensioning device 23. In Figure 1, only one tensioning device is showed on the first belt 4. This tensioning device is located on the right side of the seat pad 1. It should be understood that there could optionally be one or several additional tensioning devices on the first belt 4, for example a tensioning device on the left side of seat pad 1. The first belt 4 can be secured to the top surface of the seat pad 1. For example, the first belt 4 can be sewed to the top surface 2 of the seat pad 1. Alternatively, the first belt 4 can be removably attached to the seat pad 1. It should also be understood that the seat pad 1 may be formed by two separate elements between which the belt 4 is sandwiched.

With reference to Figures 4 and 5, the posture support device preferably also comprises a second belt 5 that can be fastened to the backrest 6 of a seat or chair 7. The ends 8, 9 of the second belt 5 can be fastened either to the first belt 4 or to the seat pad 1 (or, optionally, the cushion of a seat) such that the second belt thereby can secure the user P to the backrest of the seat or chair 7. As showed in Figures 1 - 3, the first belt 4 may be provided with straps 10, 11 fastened to the first belt 4. The straps 10, 11 and the ends 8, 9 of the second belt 5 have cooperating fasteners 12, 13, 14, 15 such that the ends 8, 9 of the second belt 5 can be locked to the straps 10, 11. The fasteners 12, 13, 14 15 may be, for example, buckles 12, 13, 14, 15, 16 of the same kind used for ordinary seat belts.

Preferably, the length of the second belt 5 can be adjusted by one or several tensioning devices 23. With reference to Figure 5, it should be noted that the second belt 5 is showed crossing itself. This way of using the belt is preferable since it will provide better comfort to the user of the posture support device, especially if the user is a woman. The reason is that the crossed belt will not cause pressure over the woman's breasts which could be uncomfortable. Additionally, if the woman is pregnant, the crossed belt ends can avoid causing pressure over her belly.

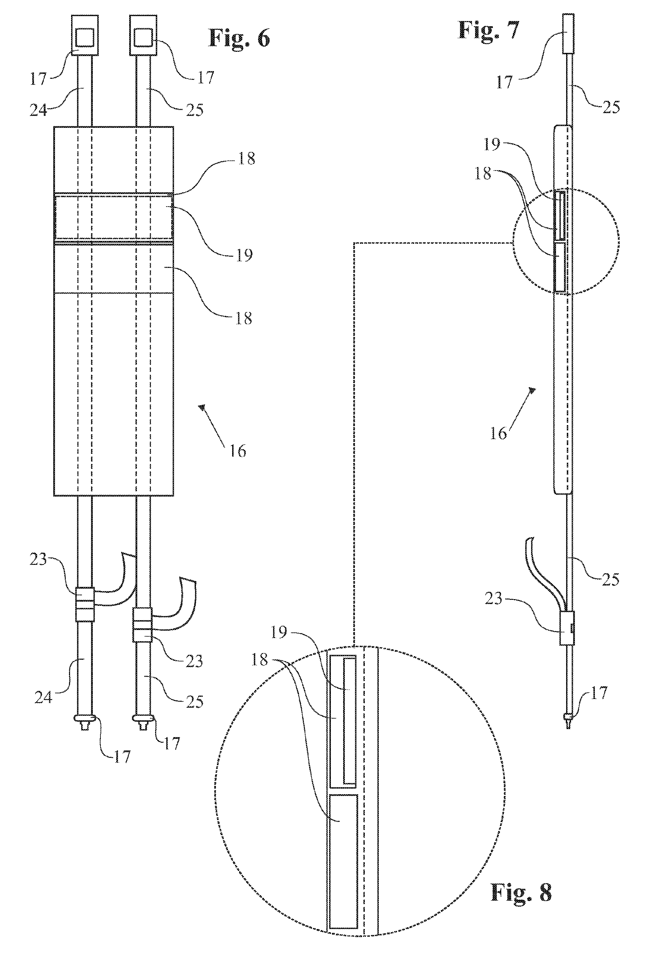

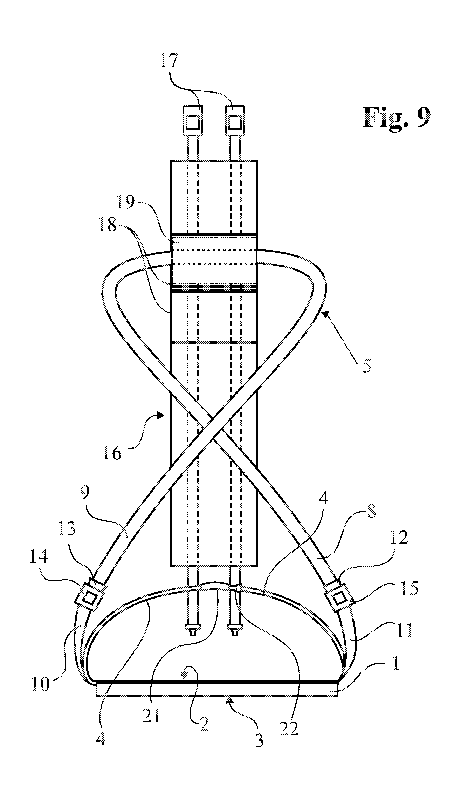

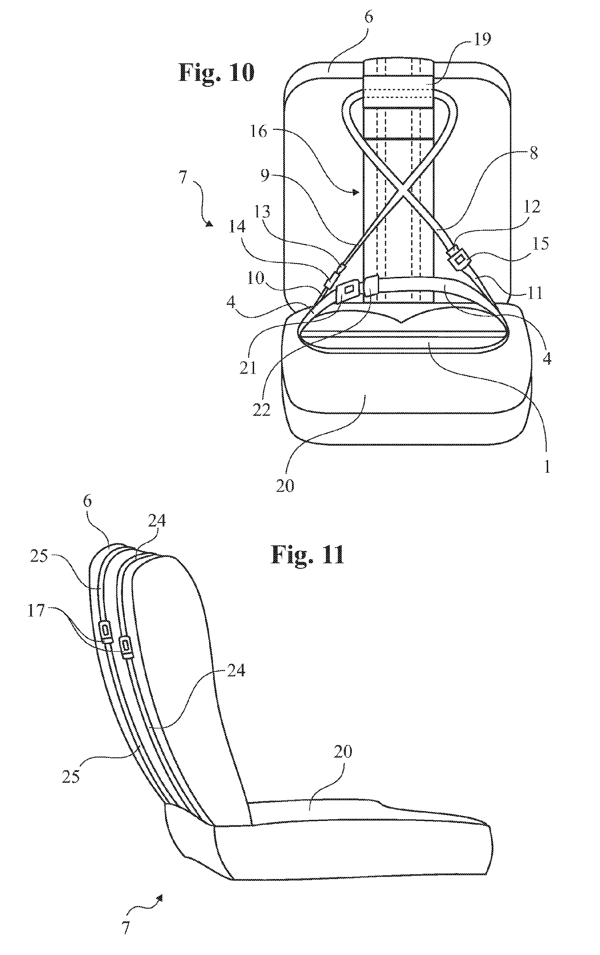

Reference will now be made to Figures 6 - 11. In an advantageous embodiment of the invention, the posture support device additionally comprises a third belt 16. The third belt 16 has means 17, for example buckles 17, for securing the third belt to the backrest 6 of a seat or chair 7 as can be seen in Figure 11. In Figures 6 and 7, the buckles 17 are showed placed at the ends of straps or belts 24, 25. In Figure 6, two straps 25, 24 are showed. However, it should be understood that it would also be possible to use only one strap or more than two straps. The length of the belts or straps 24, 25 can be adjusted by one or several tensioning devices 23. It should also be understood that it is not necessary that the straps or belts 24, 25 be connected to each other at their ends. One alternative possibility would be (for example) to connect them to hooks or hook-like elements that are integral with the backrest of the seat. Preferably, the third belt 16 is formed with at least one channel 18 through which the second belt 5 can be passed and thereby secured to the backrest 6. As best seen in Figures 6, 9 and 10, the third belt 16 may comprise a plurality of channels 18 placed such that, when the third belt is secured to the backrest 6 of a seat or chair 7, the second belt 5 can be secured to the backrest 6 at different vertical levels. In Figure 6, only two channels 18 are indicated. However, it should be understood that three, four or even more channels 18 can be used such that the second belt 5 can be placed at several different vertical levels. When the second belt 5 is secured in the third belt and crossed over the torso of the person using the posture support device, the belts 5, 16 could possibly become curved around the back of the user's neck thereby causing discomfort for the user. To prevent this, the at least one channel 18 can be provided with a stiffening insert/element 19 to prevent the third belt 16 from curving around the neck of a user P (a person supported by the posture support device) when the ends 8, 9 of the second belt 5 are fastened to the first belt 4 or the seat pad 1. The stiffening insert 19 may be, for example, a flat piece of a polymeric material that has been sewn to the third belt 16. The stiffening insert 19 can be of different widths depending on the size of the user. If the user is a child, a width of about 10 cm can be mentioned as a possible width for the stiffening element 19. A complete set of the inventive posture support device may comprise a plurality of different stiffeners 19, each stiffener having a width of its own so that the device can be adapted to different users. The stiffener 19 or stiffeners 19 may be placed in the same channel 18 through which the second belt 5 is passed. Alternatively, the stiffener 19 may be placed in a separate channel or pocket adjacent the channel 18 through which the second belt 5 is passed. It would also be possible to fasten the stiffener 19 to the third belt on for example the back side of the third belt or in a position where it can come into direct contact with the user's neck. In advantageous embodiments, the stiffener 19 or stiffeners 19 may be removably placed in different positions, e.g. in different channels 18 so that a stiffener 19 can be taken from one position and placed in another position. However, it is also conceivable that the stiffener 19 (or stiffeners 19) is in a fixed position. For example, a stiffener 19 may be sewn to the third belt 16 in a fixed position. As an example of a possible material for a stiffener 19, foamed polyvinyl chloride (PVC) can be mentioned. A stiffener 19 may thus be a flat object of foamed polyvinyl chloride having a thickness of, for example, 3 - 5 mm and a width of about 10 cm. These figures should only be understood as examples of what may be possible in one embodiment. Other dimensions and other materials are of course also possible. However, foamed polyvinyl chloride can be an advantageous material since it is possible to sew through the material itself which makes it possible to fasten the stiffener 19 to the third belt 16 in a simple way if this is desired.

Alternatively, the third belt 16 can be prevented from curving by other means. For example, the third belt 16 can be secured by buttons or other fasteners to the backrest 6 of a seat 7. It should be understood that when the third belt 16 is prevented from curving, the second belt 5 is thereby also prevented from curving around the neck of the user.

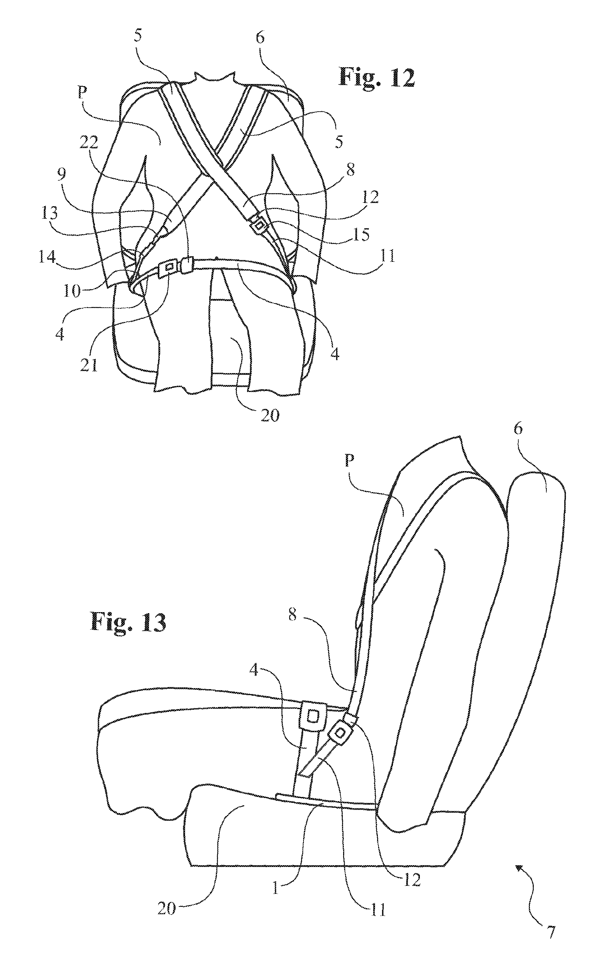

Reference will now be made to Figures 10 and 12 - 15. The invention also relates to a seat or chair 7 having a cushion 20 and a first belt 4 with adjustable length and adapted to be locked over a part of the body of a user/person P sitting on the seat or chair 7 such that the first belt 4 locks over the body below the hips of the person sitting on the seat or chair 7. Tightening of the first belt 4 will thereby secure the rear of the user's body either against the cushion 20 of the seat or chair 7 or against a seat pad 1 associated with the first belt 4. In the embodiment showed in Figure 10 and 13, the seat or chair 7 uses a seat pad 1 of the kind described with reference to Figures 1 - 3. When a person or user is sitting on the seat or chair 7 and the first belt 4 is tightened, this will secure the seat pad 1 against the rear or bottom of the person sitting in the seat or chair 7. The user's own weight will further press the seat pad 1 against the cushion 20 of the seat 7. Since the bottom surface 3 of the seat pad 1 is a high friction surface, the seat pad 1 will not slide or slip relative to the cushion 20. Hence, there is no risk that the user will slide downwards in the seat. Optionally, instead of relying entirely on friction between the seat pad 1 and the cushion 20, the seat pad 1 can be secured to the cushion 20 of a seat or chair 7 by special fastening means such as a belt, buttons, hooks or other separate fasteners. The first belt 4 is applied over the user's body below the hips of the user. This makes it easier to secure the user against sliding. A belt applied above the hips entails a greater risk that the user can slide under the belt.

Preferably, the seat or chair 7 additionally comprises a backrest 6 and second belt means 5 fastened to the backrest 6. The second belt means 5 is adapted to be fastened to either the cushion 20 or to the first belt 4 such that the torso of a user/person P sitting in the seat or chair 7 is secured against the backrest 6.

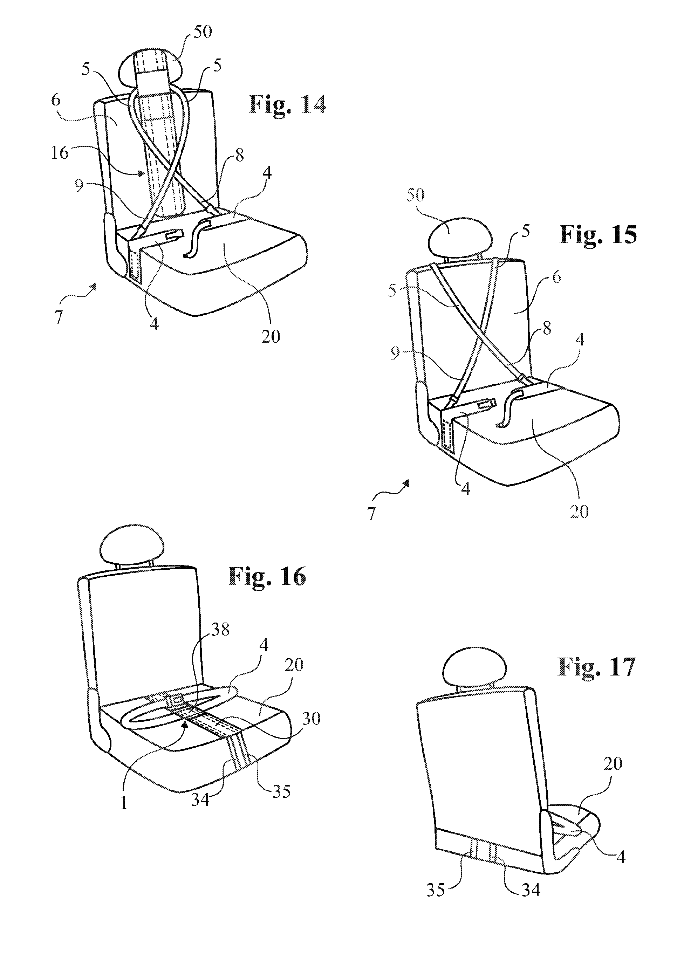

The second belt means 5 could comprise such a belt 5 that is described with reference to Figures 4 and 5 and is removably attached to the backrest 6 of the seat or chair 7 by means of such a third belt 16 that is described with reference to Figures 6 - 8 and having channels 18 with stiffening inserts 19. However, the second belt means 5 can also be straps or belts 5 integral with the backrest 6. Such an embodiment is indicated in Figure 15.

It should be noted that Figures 14 and 15 also show embodiments where there is no separate seat pad 1 as in Figures 10 and 13. Instead, the first belt 4 is integral with the cushion 20. In Figures 14 and 15, the first belt 4 is showed secured (e.g. sewn) to the sides of the seat cushion 20. Thereby, it is possible to tighten the first belt 4 over a part of the user's body that is below the hips and press the user's body against the seat cushion 20 thereby reliably securing the user P against sliding downwards on the seat cushion 20. With reference to Figure 14, it should also be noted that an alternative way of securing the second belt 5 to the backrest 6 is to pass the second belt 5 in a loop around a part of the seat 7. In Figure 14, the second belt 5 is simply passed behind a headrest or head restraint 50 instead of being locked in a channel 18 of the third belt. In this embodiment, it is the headrest 50 that prevents the second belt 5 from curving around the neck of the user P when the ends 8, 9 of the belt 5 cross each other over the user's chest.

With reference to Figure 15, it is of course the backrest 6 itself that prevents the second belt means 5 from curving around the neck of the user P since the upper ends of the belts 5 are integral with the backrest 6. This can be achieved, for example, by sewing the upper ends of the belts 5 to an upper part of the backrest at points spaced apart from each other in the horizontal direction. Instead of sewing the upper ends of the belts 5 to the backrest 6, the upper ends of the belts 5 can be fastened to the backrest 6 by other means, for example buttons or hooks.

A suitable way of using the invention can be as follows. The third belt 16 is locked to a backrest 6 of a seat or chair 7 by means of the buckles 17 or by other means suitable for locking the third belt to the backrest 6. By means of the tensioning devices 23, the straps or belts 24, 25 can be shortened until the third belt is tightly secured to the backrest 6 or seatback 6. The second belt 5 is secured in one of the channels 18. The seat pad 1 is placed on the cushion 20. The user can now be positioned sitting on the seat pad 1. The first belt 4 is locked over the user's body and the first belt is tightened so that the user's body is secured to the seat pad 1. The second belt 5 is crossed over itself as indicated in Figure 5, 9, 10 and 12. The ends 8, 9 of the second belt 5 are locked to the straps 10, 11 of the first belt 4 by means of the fasteners 12, 13, 14, 15. The second belt 5 can then be tightened by means of the tensioning devices 23.

An alternative embodiment of the invention is disclosed in Figure 16. In Figure 16, the first belt 4 is secured to the cushion by means of a fourth belt 30 similar in design to the third belt 16. The fourth belt 30 may have one or several channels 38 similar to the channel or channels 18 of the third belt 16. The first belt 4 may be passed through a channel 38 of the fourth belt. The fourth belt, in turn, is secured to the cushion 20 by means of fastening means such as buckles or hooks. The fourth belt 30 may have one or several tensioning devices to help securing the fourth belt to the cushion 20. A frictional seat pad 1 may be fixed to the fourth belt 30, e.g. by sewing. In Figure 16, the fourth belt 30 is showed extending in a direction perpendicular to the loop of the first belt 4. It should be understood that it could also be turned 90 degrees. In that case, the orientation of the channel or channels 38 of the fourth belt 30 would also have to be turned in a corresponding way to accommodate the first belt 4. It should also be understood that, instead of using channels 38, the first belt 4 could be secured to the fourth belt 30 to make the belts integral with each other. For example, the first belt 4 could be sewed to the fourth belt 30.

It should be understood that the invention also relates to a vehicle comprising such a seat or chair as described above with reference to Figures 10 - 11 or equipped with such a posture support device as described with reference to Figures 1 - 9 and 16 - 17.

It should also be understood that the invention described is not a safety belt and is not intended to provide security in case of a collision between vehicles. When the invention is used in a vehicle, it would be suitable to use an ordinary safety belt in addition to the posture support device.

While the invention has been explained with reference to use in a vehicle, e.g. a bus or car, it should be understood that in can also be used in other contexts. For example, the invention can be applied to wheelchairs, chairs in an office or to a chair or sofa in the user's own home or at public places, e.g. restaurants or libraries.

The idea of using stiffening inserts in the channels 18 of the third belt can be used independently of the first belt 4 and the seat pad 1. In the same way, the more general idea of providing means for preventing the second belt from curving around the neck of the user can be used independently of whether a separate first belt 4 and a seat pad 1 is used or independently of how such equipment is designed.

The invention can also be understood in terms of a method for securing a physically impaired person in a seat, for example a vehicle seat. Such a method would include the steps that would be the natural result of using the above described equipment for securing a person to a vehicle seat. For example, the method could include the steps of placing a frictional seat pad on a vehicle seat, placing a person on the seat pad, locking and tightening the first belt 4 over the person's body below the hips and optionally applying the second belt 5 secured to the backrest 6 of the seat. The ends of the second belt 5 can then be crossed and fastened. For example, the ends of the second belt 5 can be fastened to straps 10, 11 on the first belt.

The second belt means (e.g. a single belt or a pair of belts) are prevented from curving around the user's neck.

While the invention has been explained in terms of a device for posture support, a seat or chair, a vehicle and a method for securing a person, it should be understood that these different categories only reflect different aspects of one and the same invention. For example, a seat or chair according to the present invention may be the result of the inventive posture support device being applied to a vehicle seat.

## Claims

1. A device for providing posture support to a seated user (P), the device comprising
a) a seat pad (1) with a top surface (2) and a bottom surface (3),
b) a first belt (4) attached to or attachable to the seat pad, the first belt being adapted to locked into a loop such that the first belt can loop a part of the user's body when the user is sitting on the top surface of the seat pad (1) and thereby secure the rear of the user to the seat pad (1),
c) a second belt (5) that can be fastened to the backrest (6) of a seat or chair (7), the device further being adapted to fasten ends (8, 9) of the second belt either to the first belt (4) or to the seat pad (1) such that the second belt thereby can secure the user (P) to the backrest of the seat or chair (7) and
d) means for preventing the second belt (5) from curving around the neck of a user (P) when the second belt (5) is crossed over the torso of a user (P).

2. A device according to claim 1, wherein the first belt (4) is provided with straps (10, 11) and wherein the straps (10, 11) and the ends (8, 9) of the second belt (5) have cooperating fasteners (12, 13, 14, 15) such that the ends (8, 9) of the second belt (5) can be locked to the straps (10, 11).

3. A device according to claim 1 or claim 2, wherein the device additionally comprises a third belt (16) having means (17) for securing the third belt to the backrest (6) of a seat or chair (7), the third belt being formed with at least one channel (18) through which the second belt (5) can be passed and thereby secured to the backrest (6).

4. A device according to claim 3, wherein the third belt (16) comprises a plurality of channels (18) placed such that, when the third belt is secured to the backrest (6) of a seat or chair (7), the second belt (5) can be secured to the backrest (6) at different vertical levels.

5. A device according to claim 3, wherein the at least one channel (18) is provided with a stiffening insert (19) to prevent the third belt (16) from curving around the neck of a user (P) when the ends (8, 9) of the second belt (5) are fastened to the first belt (4) or the seat pad (1).

6. A device according to claim 2, wherein the lengths of the first belt (4) and the second belt (5) can be adjusted.

7. A seat or chair (7) having a cushion (20) and a first belt (4) with adjustable length and adapted to be locked over a part of the body of a user (P) sitting on the seat or chair (7) such that the first belt (4) locks over the body below the hips of the user sitting on the seat or chair (7), whereby tightening of the first belt will secure the rear of the user's body against the cushion (20) of the seat or chair (7) or against a seat pad (1) associated with the first belt (4) and wherein the seat or chair (7) additionally comprises a backrest (6) and second belt means (5) fastened to the backrest (6), the second belt means (5) being adapted to be fastened to either the cushion (20) or to the first belt (4) such that the torso of a user (P) sitting in the seat or chair (7) is secured against the backrest (6) and wherein means are provided for preventing the second belt means (5) from curving around the neck of a user (P) when the second belt means (5) is crossed over the torso of a user.

8. A seat or chair (7) according to claim 7, wherein the second belt means (5) comprises a belt (5) that is removably attached to the backrest (6) of the seat or chair (7).

9. A seat or chair (7) according to claim 7, wherein the second belt means (5) comprises straps (5) integral with the backrest (6).

10. A vehicle comprising a vehicle seat according to any of claims 8 - 9.

11. A method for securing a physically impaired person in a seat (7) having a backrest (6), e.g. a vehicle seat, the method comprising the steps of:
a) placing the person on the seat (7),
b) locking a first belt (4) over the persons body below the hips,
c) using second belt means (5) secured to the backrest (6) and applying the second belt means (5) such that ends of the second belt means (5) cross each other over the torso of the person (P) sitting on the seat and
d) providing means (19, 50) for preventing the second belt means (5) from curving around the neck of the person (P) sitting on the seat (7).

12. A method according to claim 11 wherein the second belt means (5) comprises a belt (5) which is secured to the backrest (6) by a third belt (16) having at least one channel (18) through which the second belt (5) is passed and wherein the means (19, 50) for preventing the second belt (5) from curving around the neck of the person sitting on the seat comprises a stiffening element (19) in the at least one channel (18) of the second belt (5).
